# EUROPÄISCHE PATENTANMELDUNG

(11) **EP 2 080 477 A2**
(43) Veröffentlichungstag der Anmeldung: **22.07.2009**
(21) Anmeldenummer: 08172993.1
(22) Anmeldetag: 29.12.2008
(51) Int. Cl.: A61B 6/04

(54) **Positioniervorrichtung zum Positionieren eines Patienten, Partikeltherapieanlage sowie Verfahren zum Betreiben einer Positioniervorrichtung**

(30) Priorität: 18.01.2008 DE 102008005069
(71) Anmelder: Siemens Aktiengesellschaft, 80333 München (DE)
(72) Erfinder: Hüttenberger, Stefan, 91054 Erlangen (DE); Rietzel, Eike, Dr., 64289 Darmstadt (DE)

(57) **Zusammenfassung**

Die Erfindung betrifft eine Positioniervorrichtung zum Positionieren eines Patienten in einer medizinischen Diagnostik- und/oder Therapievorrichtung, umfassend:
- eine Patientenlagerungsvorrichtung, auf der ein Patient lagerbar ist, und
- einen Roboterarm mit mehreren Bewegungsachsen, mit dem die Patientenlagerungsvorrichtung im Raum positionierbar ist,

wobei die Positioniervorrichtung in einen manuellen Betriebsmodus versetzbar ist, in welchem eine Position der Patientenlagerungsvorrichtung im Raum manuell änderbar ist. Weiterhin betrifft die Erfindung ein Verfahren zum Betreiben einer derartigen Positioniervorrichtung, umfassend:
- Bereitstellen eines regulären Betriebsmodus, in dem die Patientenlagerungsvorrichtung vollautomatisch an einer durch eine Steuerungsvorrichtung vorgegebenen Position positioniert wird,
- Bereitstellen eines manuellen Betriebsmodus, in dem die Position der Patientenlagerungsvorrichtung im Raum manuell änderbar ist,
- Wechsel von dem regulären Betriebsmodus in den manuellen Betriebsmodus bei Vorliegen einer Wechselbedingung.

Weiterhin betrifft die Erfindung eine Bestrahlungsvorrichtung mit einer derartigen Positioniervorrichtung.

## Beschreibung

Die Erfindung betrifft eine Positioniervorrichtung zum Positionieren eines Patienten in einer medizinischen Diagnostik- und/oder Therapieanlage, wie sie insbesondere in einer Partikeltherapieanlage zum Positionieren eines Patienten relativ zu einem Behandlungsstrahl eingesetzt wird. Weiterhin betrifft die Erfindung eine Partikeltherapieanlage mit einer derartigen Positioniervorrichtung sowie ein Verfahren zum Betreiben einer derartigen Positioniervorrichtung.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden üblicherweise geladene Partikel auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit dem Partikelstrahl bestrahlt. Hierbei ist es für den Erfolg einer Bestrahlung wesentlich, dass das zu bestrahlende Objekt möglichst genau relativ zu dem Partikelstrahl positioniert wird.

Es sind Vorrichtungen bekannt, bei denen die Positionierung beispielsweise eines Patienten mit einer roboterbasierten Positioniervorrichtung erfolgt. Beispielsweise kann eine Patientenliege durch einen mehrachsigen Roboterarm flexibel relativ zu einem Partikelstrahl positioniert werden.

Es ist daher die Aufgabe der Erfindung, eine Positioniervorrichtung zum Positionieren eines Patienten in einer medizinischen Diagnostik- und/oder Therapievorrichtung anzugeben, mit der ein hohes Maß an Patientensicherheit während des Positionierens des Patienten gewährleistet ist. Weiterhin ist es die Aufgabe der Erfindung, eine Bestrahlungsvorrichtung anzugeben, die ein hohes Maß an Patientensicherheit während des Positionierens des Patienten gewährleistet sowie ein Verfahren zum Betreiben einer derartigen Positioniervorrichtung.

Die Aufgabe der Erfindung wird gelöst durch eine Positioniervorrichtung nach Anspruch 1, durch eine Bestrahlungsvorrichtung nach Anspruch 8 sowie durch ein Verfahren zum Betreiben einer Positioniervorrichtung nach Anspruch 10. Vorteilhafte Weiterbildungen finden sich in den Merkmalen der unabhängigen Ansprüche.

Die erfindungsgemäße Positioniervorrichtung zum Positionieren eines Patienten in einer medizinischen Diagnostik- und/oder Therapievorrichtung umfasst:
- eine Patientenlagerungsvorrichtung, auf der ein Patient lagerbar ist, und
- einen Roboterarm mit mehreren Bewegungsachsen, mit dem die Patientenlagerungsvorrichtung im Raum positionierbar ist,
wobei die Positioniervorrichtung in einen manuellen Betriebsmodus versetzbar ist, in welchem eine Position der Patientenlagerungsvorrichtung im Raum manuell änderbar ist.

Der Erfindung liegt die Idee zu Grunde, dass bei bekannten Positioniervorrichtungen mit einem mehrachsigen Roboterarm zwar eine genaue und präzise Positionierung eines Patienten möglich ist, indem die Steuerung der Positioniervorrichtung üblicherweise automatisch von einer Steuereinheit übernommen wird, ggf. durch Interaktion mit einem Anwender, der die Steuerung mittels eines Handbedienteils steuert. In Notsituationen jedoch kann durch eine derartige Positioniervorrichtung der Zugang zu einem Patienten erschwert sein. In einer Notsituation, beispielsweise bei einer Fehlfunktion der Positioniervorrichtung und/oder der Anlage, in der die Positioniervorrichtung betrieben wird, wird der Roboterarm üblicherweise blockiert, so dass eine Bewegung des Patienten nicht mehr möglich über die Steuerungseinheit möglich ist. Hierdurch wird gewährleistet, dass in einer derartigen Notsituation der Roboterarm keine fehlerhaften Bewegungen ausführen kann, die die Sicherheit eines Patienten stark gefährden würden.

Eine derartige Notsituation kann beispielsweise auftreten, wenn in einer Partikeltherapieanlage ein Not-Stop betätigt wurde, wodurch einerseits der Partikelstrahl und andererseits die Stromversorgung vieler Komponenten im Behandlungsraum abgeschaltet werden. Eine derartige Notsituation kann aber auch auftreten, wenn eine Komponente einer Partikeltherapieanlage, beispielsweise die Positioniervorrichtung selbst, eine Abweichung von einer regulären Funktionsweise detektiert, und dementsprechend in einen Modus übergeführt wird, bei dem die Funktionsweise der Komponente und/oder weiterer Komponenten eingeschränkt wird, beispielsweise durch Abschalten der Stromversorgung.

Es wurde dabei erkannt, dass bei Auftreten einer Notsituation das zumindest teilweise Abschalten einzelner und/oder mehrere Komponenten vorteilhaft ist, dass hierdurch aber die Sicherheit eines Patienten gefährdet werden kann, da durch ein Blockieren des Roboterarms der Positioniervorrichtung der Patient ggf. in eine Position verharrt, in der eine Intervention am Patienten erschwert oder gar unmöglich gemacht wird. Wenn die Positioniervorrichtung beispielsweise in einem Gantry-basierten Bestrahlungsraum verwendet wird, kann - je nach Ausbildung des Bestrahlungsraums - der Bestrahlungsraum einen beweglichen Boden aufweisen, der zur Bewegung der Gantry aus dem Bestrahlungsraum gefahren wird. In diesem Zustand verfügt der Bestrahlungsraum über keinen Boden, den ein Anwender gefahrlos begehen könnte. Wenn zu einem derartigen Zeitpunkt eine Notsituation auftritt, kann beispielsweise die Positioniervorrichtung den Patienten in einer Position blockieren, in der ein Zugang zu den Patienten unmöglich ist. Doch auch in Bestrahlungsräumen mit vorhandenem Boden kann der Patient in einer Notsituation in einer derart ungünstigen Position blockiert werden, dass ein Zugang zu dem Patienten unmöglich ist, beispielsweise um Reanimationsmaßnahmen durchführen zu können.

Mit der erfindungsgemäßen Positioniervorrichtung wird eine einfache und effektive Lösung für dieses Problem bereitgestellt. Die Positioniervorrichtung ist derart ausgebildet, dass sie in einen manuellen Betriebsmodus versetzt werden kann, in welchem eine Position der Patientenlagerungsvorrichtung im Raum zumindest teilweise manuell geändert werden kann. Auf diese Weise ist es auch in Notsituationen nun möglich, die Position des Patienten im Raum manuell zu ändern, so dass ein Patient in eine für eine Bergung des Patienten bzw. Behandlung des Patienten günstigere Position gebracht werden kann.

Die Patientenlagerungsvorrichtung kann z.B. als Patientenliege oder Patientenstuhl ausgebildet sein, auf der ein Patient in einer für die Bestrahlung vorgesehene Haltung positioniert werden kann.

In einer vorteilhaften Ausführungsform der Erfindung ist die Positioniervorrichtung derart ausgebildet, dass in dem manuellen Betriebsmodus eine Rotation der Patientenlagerungsvorrichtung um eine vertikale Achse manuell ausgeführt werden kann. Dies kann bei beispielsweise dadurch realisiert werden, dass bei dem mehrachsigen Roboterarm eine Achse oder mehrere Achsen mit jeweils vertikaler Drehachse entsperrt bzw. freigegeben werden. Eine Rotation um eine vertikale Drehachse reicht üblicherweise aus, um einen auf der Patientenlagerungsvorrichtung gelagerten Patienten aus einer Gefahrensituation zu bergen. Die Entsperrung einer Achse (oder mehrere Achsen) kann auf einfache Weise z.B. durch gezielte Entblockierung der Bremsen einer Achse in dem manuellen Betriebsmodus umgesetzt werden.

In einer Ausführungsform ist die Positioniervorrichtung derart ausgebildet, dass der manuelle Betriebsmodus automatisch aktiviert wird, wenn eine Fehlerbedingung während eines regulären Betriebs der Positioniervorrichtung und/oder der Diagnostik- und/oder Therapieanlage auftritt. Eine Fehlerbedingung deutet auf eine Fehlfunktion hin, so dass in diesem Fall üblicherweise verschiedene Schritte durchgeführt werden, um die Sicherheit des Patienten zu gewährleisten. Bei einer Partikeltherapieanlage kann beispielsweise der Partikelstrahl abgeschaltet werden, um einen Bestrahlungsvorgang zu stoppen. Bei dieser Ausführungsform wird nun zusätzlich die Positioniervorrichtung automatisch in den manuellen Betriebsmodus versetzt, so dass eine manuelle Bewegung der Patientenlagerungsvorrichtung und damit des Patienten bei Vorliegen einer Fehlerbedingung stets ermöglicht ist.

In einer alternativen und/oder zusätzlichen Ausführungsform weist die Positioniervorrichtung eine manuell betätigbare Umschaltvorrichtung auf. Ein Betätigen der Umschaltvorrichtung versetzt die Positioniervorrichtung in den manuellen Betriebsmodus. Hierdurch ist es möglich, den manuellen Betriebsmodus der Positioniervorrichtung durch manuelle Intervention zu aktivieren. Auf diese Weise kann ein Patient schneller in eine gewünschte Position gebracht werden, als es beispielsweise bei einer regulären, automatisch gesteuerten Bewegung der Positioniervorrichtung im Betrieb möglich wäre. Eine reguläre Bewegung der Positioniervorrichtung ist nämlich oftmals an die Bewegung anderer Komponenten im Behandlungsraum gekoppelt, so dass bei einer automatisch gesteuerten Bewegung der Positioniervorrichtung einzelne Bewegungen aufeinander abgestimmt werden müssen, was oftmals erhebliche Zeit in Anspruch nimmt. Durch eine manuelle Betätigung der Umschaltvorrichtung kann bei Bedarf in den manuellen Betriebsmodus gewechselt werden, so dass ein Patient manuell schnell bewegt werden kann.

Dies kann beispielsweise dadurch geschehen, dass ein an der Positioniervorrichtung angeordneter Knopf gedrückt wird, wodurch beispielsweise eine bestimmte Achse des Roboterarms entsperrt wird. Die Umschaltvorrichtung kann in einer anderen Ausführungsform derart ausgebildet sein, dass die Umschaltvorrichtung an einer der Bewegungsachsen des Roboterarms angeordnet ist, insbesondere an einer Bewegungsachse mit vertikaler Drehachse, und ein Betätigen der Umschaltvorrichtung eine direkte mechanische Entsperrung diese Bewegungsachse bewirkt. Dies ist besonders von Vorteil, da auf diese Weise eine Entsperrung der Bewegungsachse in jedem Fall möglich ist, selbst wenn die Steuerung der Positioniervorrichtung vollständig ausgefallen ist.

Zur vereinfachten Bewegung der Patientenlagerungsvorrichtung sind in einer Ausführungsform an der Patientenlagerungsvorrichtung Mittel zum manuellen Ergreifen der Patientenlagerungsvorrichtung angeordnet. Derartige Mittel können beispielsweise ein Handgriff sein oder eine Aufnahmevorrichtung, in die ein an einer Stange angeordneter Haken eingeführt werden kann. Eine Leine, die von der Patientenlagerungsvorrichtung bis zu einem Anwender reicht, ermöglicht eine Bewegung der Patientenlagerungsvorrichtung selbst dann, wenn die Patientenlagerungsvorrichtung sich nicht in direkter Reichweite eines Anwenders befindet. Die Leine kann beispielsweise fest am Roboterarm angebracht sein und sich bei Bedarf durch Ziehen lösen lassen.

Die erfindungsgemäße Bestrahlungsvorrichtung umfasst:
- eine Partikelquelle zur Erzeugung von Partikeln,
- einen Beschleuniger zur Beschleunigung der Partikel und zur Bereitstellung eines hochenergetischen Partikelstrahls,
- einen Bestrahlungsraum zur Bestrahlung eines zu bestrahlenden Objekts mit dem hochenergetischen Partikelstrahl und
- eine in dem Bestrahlungsraum angeordnete Positioniervorrichtung nach einem der Ansprüche 1 bis 7.

In einer speziellen Ausführungsform umfasst der Bestrahlungsraum eine bewegbare Gantry, so dass der Partikelstrahl aus verschiedenen einstellbaren Richtungen auf das zu bestrahlende Objekt gerichtet werden kann, sowie einen entfernbaren Boden im Bestrahlungsraum, insbesondere im Gantrybereich des Bestrahlungsraums. Anhand des entfernbaren Bodens kann zusätzlicher Bewegungsspielraum zur Bewegung der Gantry geschaffen werden. Der Einsatz der erfindungsgemäßen Positioniervorrichtung wirkt sich in einem derartigen Raum besonders vorteilhaft aus, da hier - im Falle einer Blockade der Positioniervorrichtung im Bereich des entfernten Bodens - eine Bergung eines Patienten ansonsten nur sehr aufwändig möglich wäre.

Bei dem erfindungsgemäßen Verfahren zum Betreiben einer Positioniervorrichtung, mit der ein Patient in einer medizinischen Diagnostik- und/oder Therapieanlage positioniert werden kann, wird:
- ein regulärer Betriebsmodus bereitgestellt, während dessen die Patientenlagerungsvorrichtung vollautomatisch an einer durch eine Steuerungsvorrichtung vorgegebenen Position positioniert wird,
- ein manueller Betriebsmodus bereitgestellt, während dessen die Position der Patientenlagerungsvorrichtung im Raum manuell änderbar ist, und
- einen Wechsel von dem regulären Betriebsmodus in den manuellen Betriebsmodus durchgeführt, sobald eine Wechselbedingung vorliegt.

In einer Ausführungsform kann bei dem Verfahren eine Wechselbedingung automatisch immer dann vorliegen, wenn eine Fehlerbedingung während des regulären Betriebsmodus festgestellt wird. Die Fehlerbedingung kann dabei beispielsweise eine Fehlfunktion der Positioniervorrichtung charakterisieren oder aber auch eine Fehlfunktion der medizinischen Diagnostik- und/oder Therapieanlage kennzeichnen.

In einer anderen Ausführungsform liegt die Wechselbedingung immer dann vor, wenn eine Umschaltvorrichtung manuell betätigt worden ist. Die Umschaltvorrichtung kann beispielsweise ein Not-Stopp-Schalter sein, durch dessen Betätigung eine Reihe von Vorgängen ausgelöst wird, beispielsweise eine Unterbrechung des Bestrahlungsvorgangs. Durch Betätigung der Umschaltvorrichtung wird der Wechsel von dem regulären Betriebsmodus in den manuellen Betriebsmodus durchgeführt.

Die Umschaltvorrichtung kann in einer speziellen Ausführungsform aber auch an einer Bewegungsachse des Roboterarms angeordnet sein. Insbesondere ist eine direkte mechanische Entsperrung der Bewegungsachse des Roboterarms durch Betätigen der Umschaltvorrichtung möglich.

Ausführungsformen der vorliegenden Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: einen schematischen Überblick über eine Partikeltherapieanlage,
- Fig. 2: eine perspektivische Ansicht eines Gantry-basierten Bestrahlungsraums mit einer Positioniervorrichtung,
- Fig. 3: eine Übersicht über Verfahrensschritte, die beim Betreiben der Positioniervorrichtung durchgeführt werden.

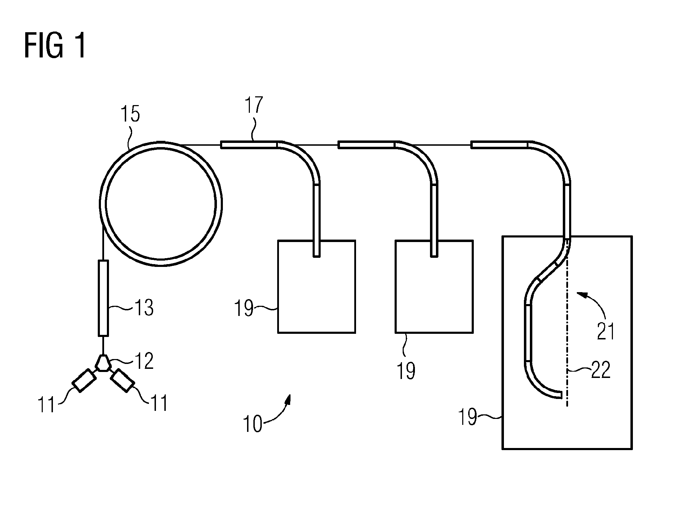

Figur 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen; beispielsweise muss, je nach Beschleunigung der Partikel, eine Bestrahlungsvorrichtung nicht als Partikeltherapieanlage angeordnet sein.

Die nachfolgend beschriebenen Ausführungsbeispiele sind sowohl in Zusammenhang mit der anhand von Fig. 1 dargestellten Partikeltherapieanlage als auch mit anderen Partikeltherapieanlagen oder Strahlentherapieanlagen einsetzbar.

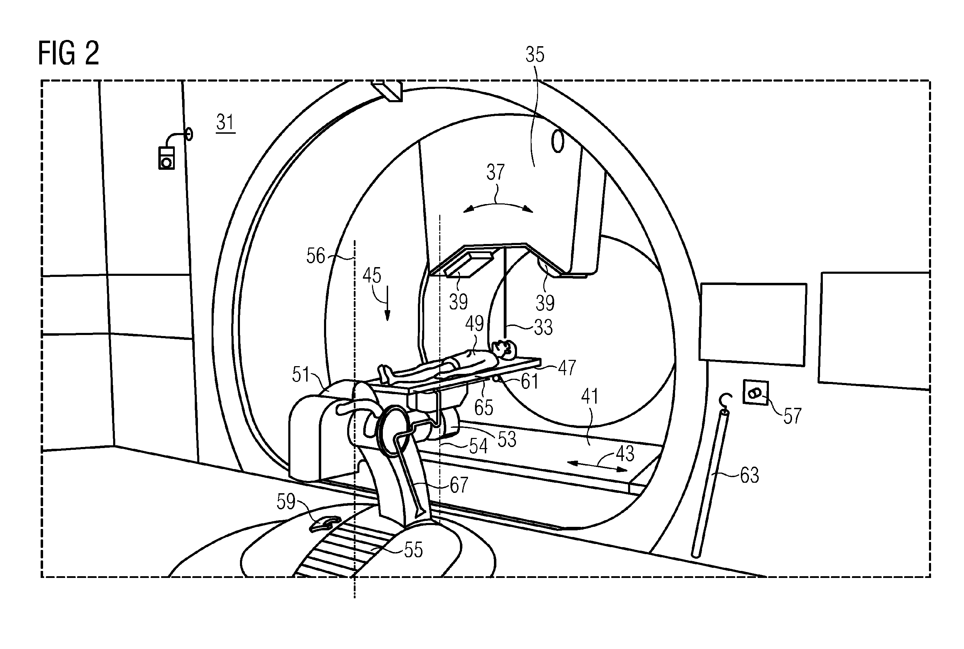

Fig. 2 zeigt eine perspektivische Ansicht eines Gantry-basierten Bestrahlungsraums 31, in dem eine Positioniervorrichtung 45 zum Positionieren eines Patienten 49 eingesetzt wird.

Die Bestrahlung des Patienten 49 erfolgt mit einem Partikelstrahl 33, der aus einer Partikelstrahlabgabevorrichtung 35 austritt. Die Partikelstrahlabgabevorrichtung 35 ist an einer in Fig. 2 nicht sichtbaren Gantry angeordnet, die es ermöglicht, die Partikelstrahlabgabevorrichtung 35 derart im Bestrahlungsraum zu rotieren (Doppelpfeil 37), dass der Partikelstrahl 33 aus mehreren Winkeln abgegeben werden kann. An der gezeigten Partikelstrahlabgabevorrichtung 35 sind weiterhin Flachbilddetektoren 39 angeordnet, die zur Positionsüberprüfung der Position des Patienten 49 im Bestrahlungsraum 31 eingesetzt werden können. Um die Partikelstrahlabgabevorrichtung 35 in einem Winkelbereich von vorzugsweise 0° bis 360° positionieren zu können, kann ein Teil des Bodens 41 im Bestrahlungsraum 31 seitlich aus dem Bestrahlungsraum 31 gefahren werden (gerader Doppelpfeil 43). Hierdurch wird Platz für die Rotation der Gantry geschaffen, so dass die Partikelstrahlabgabevorrichtung 35 beispielsweise auch so positioniert werden kann, dass der Partikelstrahl 33 von unten auf einen Patienten 49 gerichtet wird.

Die Positioniervorrichtung 45 ist als mehrachsiger Roboterarm 51 ausgebildet, mit dem eine Patientenlagerungsvorrichtung 47, hier als Patientenliege gezeigt, im Bestrahlungsraum 31 positioniert werden kann. Anstelle einer Patientenliege 47 kann beispielsweise auch ein Patientenstuhl eingesetzt werden, auf dem der Patienten 49 in sitzender Haltung gelagert werden kann. Nicht gezeigt ist eine Steuerungsvorrichtung, mit der die Positioniervorrichtung 45 betrieben wird, beispielsweise um einen Patienten 49 automatisch an eine vordefinierte Position zu fahren.

Eine erste Bewegungsachse 53 des Roboterarms 51 ermöglicht es, die Patientenlagerungsvorrichtung 47 um eine erste vertikale Drehachse 54 zu rotieren. Eine zweite Bewegungsachse 55 ermöglicht es, die Patientenlagerungsvorrichtung 47 um eine zweite vertikale Drehachse 56 zu rotieren. Die erste Bewegungsachse 53 ist dabei unmittelbar unterhalb der Patientenlagerungsvorrichtung 47 angeordnet, während die zweite Bewegungsachse 55 am Boden des Bestrahlungsraums 31 angeordnet ist. Eine Drehung um die erste vertikale Drehachse 54 dreht lediglich die Patientenlagerungsvorrichtung 47. Eine Drehung um die zweite vertikale Drehachse 56 dreht den gesamten Roboterarm 51 mit der Patientenlagerungsvorrichtung 47 um die zweite vertikale Drehachse 56.

Die Positioniervorrichtung 45 ist derart ausgebildet, dass sie in einen manuellen Betriebsmodus versetzt werden kann, der es erlaubt, eine Rotation der Patientenlagerungsvorrichtung 47 um die erste vertikale Drehachse 54 und/oder um die zweite vertikale Drehachse 56 manuell durchzuführen. Dies kann beispielsweise dadurch ausgeführt werden, indem ein nicht gezeigter Anwender einen Handgriff 65 der Patientenlagerungsvorrichtung 47 ergreift und Kraft auf die Patientenlagerungsvorrichtung 47 ausübt. Alternativ und/oder zusätzlich kann ein Anwender mit einer Stange 63, die einen Haken aufweist, eine Öse 61 der Patientenlagerungsvorrichtung 47 ergreifen und Kraft auf die Patientenlagerungsvorrichtung 47 ausüben. Eine weitere Möglichkeit ist es, eine am Roboterarm 51 angeordnete Leine 67 zu lösen und über die Leine 67 Zug auf die Patientenlagerungsvorrichtung 47 auszuüben.

Hierdurch lässt sich in einer Notsituation der Patient 49 mit der Patientenlagerungsvorrichtung 47 derart bewegen, dass er manuell in eine Position gebracht werden kann, in der eine Versorgung des Patienten 49 besser und einfacher durchgeführt werden kann. Dies ist insbesondere dann der Fall, wenn der Boden 41 des Gantry-basierten Bestrahlungsraums 31 aus dem Bestrahlungsraum 31 gefahren ist, was ein direktes Zugehen auf den Patienten 49, der im Bereich der Partikelstrahlabgabevorrichtung 35 positioniert ist, unmöglich macht. Insbesondere, wenn eine Notsituation auftritt, beispielsweise wenn ein Not-Stopp-Knopf 57 gedrückt wurde, mit dem sowohl ein Bestrahlungsvorgang abgebrochen wird als auch eine automatische Steuerung der Position der Partikelstrahlabgabevorrichtung 35 und der Positioniervorrichtung 47 abgeschaltet wird, da ein unvorhergesehenes Ereignis aufgetreten ist, kann auf einfache Weise eine Bergung des Patienten 49 aus einem Gefahrenbereich manuell erfolgen, ohne dass das System zuvor wieder gestartet werden muss, um eine automatische Steuerung der Positioniervorrichtung 47 durchzuführen.

Hierzu können die erste Bewegungsachse 53 und/oder die zweite Bewegungsachse 55 des Roboterarms 51 beispielsweise automatisch freigegeben werden, wenn der Not-Stopp-Knopf 57 gedrückt wurde. Alternativ und/oder zusätzlich kann eine Freigabe einer Bewegungsachse durch direktes Betätigen eines Hebels, der an der Bewegungsachse angeordnet ist, erfolgen. In Fig. 2 ist beispielsweise an der zweiten Bewegungsachse 55 ein Hebel 59 angeordnet, durch dessen Betätigung die Bewegungsachse 55 direkt mechanisch entsperrt werden kann, so dass eine Bewegung des Roboterarms um diese Bewegungsachse manuell ermöglicht wird.

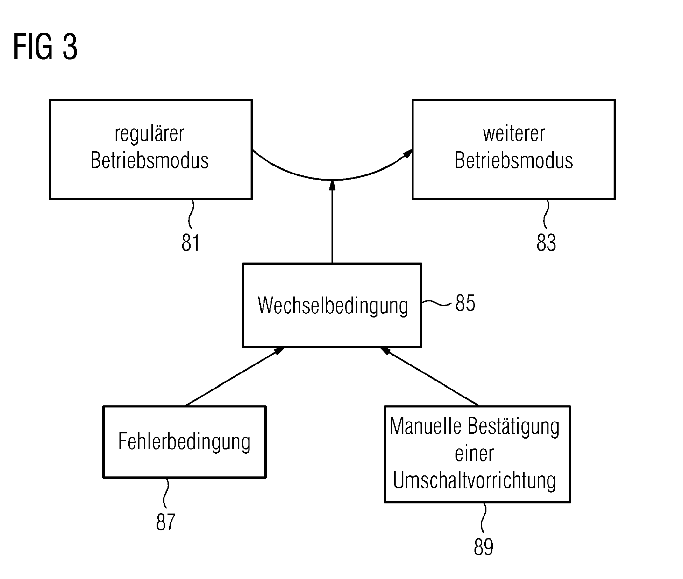

Fig. 3 zeigt eine schematische Übersicht über Verfahrensschritte, die beim Betreiben der Positioniervorrichtung durchgeführt werden.

Die Positioniervorrichtung kann in einem regulären Betriebsmodus 81 betrieben werden, bei dem eine vollständig automatische Positionierung eines Patienten mit der Positioniervorrichtung durchgeführt wird. Eine manuelle Positionierung der Positioniervorrichtung ist in dem regulären Betriebsmodus 81 nicht möglich. Weiterhin kann die Positioniervorrichtung in einem manuellen Betriebsmodus 83 betrieben werden, bei dem eine manuelle Positionierung der Positioniervorrichtung, insbesondere der Patientenlagerungsvorrichtung, im Raum ermöglicht wird.

Während des Betreibens der Positioniervorrichtung kann von dem regulären Betriebsmodus 81 in den manuellen Betriebsmodus 83 umgeschaltet werden, sobald eine Wechselbedingung 85 vorliegt. Je nach Ausgestaltung der Positioniervorrichtung kann eine derartige Wechselbedingung 85 automatisch dann vorliegen, wenn beim Betreiben der Positioniervorrichtung eine Fehlerbedingung 87 festgestellt wurde, beispielsweise durch eine Fehlfunktion der Positioniervorrichtung selbst oder durch eine Fehlfunktion der Diagnostik- und/oder Therapieanlage, in der die Positioniervorrichtung betrieben wird.

Eine Wechselbedingung 85 kann alternativ und/oder zusätzlich auch dann vorliegen, wenn eine manuelle Betätigung 89 einer Umschaltvorrichtung durchgeführt wurde. Eine derartige Umschaltvorrichtung kann beispielsweise der in Fig. 2 gezeigte Not-Stopp-Knopf 57 sein, durch dessen Betätigung manuell die Wechselbedingung ausgelöst wurde, wodurch die Positioniervorrichtung in den manuellen Betriebsmodus versetzt wird, oder beispielsweise ein Hebel 59, der gezogen werden kann.

## Patentansprüche

1. Positioniervorrichtung zum Positionieren eines Patienten (49) in einer medizinischen Diagnostik- und/oder Therapievorrichtung (10), umfassend:
- eine Patientenlagerungsvorrichtung (47), auf der ein Patient (49) lagerbar ist, und
- einen Roboterarm (51) mit mehreren Bewegungsachsen (53, 55), mit dem die Patientenlagerungsvorrichtung (47) positionierbar ist,
wobei die Positioniervorrichtung (45) in einen manuellen Betriebsmodus (83) versetzbar ist, in welchem eine Position der Patientenlagerungsvorrichtung (47) manuell änderbar ist.

2. Positioniervorrichtung nach Anspruch 1, wobei
in dem manuellen Betriebsmodus (83) eine Rotation der Patientenlagerungsvorrichtung (47) um eine vertikale Drehachse (54, 56) ausführbar ist.

3. Positioniervorrichtung nach Anspruch 1 oder 2, wobei
in dem manuellen Betriebsmodus (83) zumindest eine der Bewegungsachsen (53, 55) entsperrt ist, so dass der Roboterarm (51) um die zumindest eine Bewegungsachse (53, 55) manuell bewegbar ist.

4. Positioniervorrichtung nach einem der Ansprüche 1 bis 3, wobei
die Positioniervorrichtung (45) derart ausgebildet ist, dass der manuelle Betriebsmodus (83) automatisch aktiviert wird, wenn eine Fehlerbedingung (87) während eines regulären Betriebs der Positioniervorrichtung (45) und/oder der Diagnostik- und/oder Therapieanlage (10) auftritt.

5. Positioniervorrichtung nach einem der Ansprüche 1 bis 4, wobei
die Positioniervorrichtung (45) eine manuell betätigbare Umschaltvorrichtung (57, 59) aufweist, die derart ausgebildet ist, dass eine Betätigung der Umschaltvorrichtung (57,59) die Positioniervorrichtung (45) in den manuellen Betriebsmodus (83) versetzt.

6. Positioniervorrichtung nach Anspruch 5, wobei
die Umschaltvorrichtung (59) an einer der Bewegungsachsen (55) des Roboterarms (51) angeordnet ist und derart ausgebildet ist, dass eine Betätigung der Umschaltvorrichtung (59) die Bewegungsachse (55) des Roboterarms (51) mechanisch entsperrt.

7. Positioniervorrichtung nach einem der Ansprüche 1 bis 6, wobei
an der Patientenlagerungsvorrichtung (47) Mittel (61, 65, 67) zum manuellen Ergreifen und Bewegen der Patientenlagerungsvorrichtung angeordnet sind.

8. Bestrahlungsvorrichtung, umfassend:
- eine Partikelquelle (11) zur Erzeugung von Partikeln,
- einen Beschleuniger (13, 15) zur Beschleunigung der Partikel und zur Bereitstellung eines hochenergetischen Partikelstrahls,
- einen Bestrahlungsraum (19, 31) zur Bestrahlung eines zu bestrahlenden Objekts (49) mit dem hochenergetischen Partikelstrahl, und
- eine in dem Bestrahlungsraum (19, 31) angeordnete Positioniervorrichtung (45) nach einem der Ansprüche 1 bis 7.

9. Bestrahlungsvorrichtung nach Anspruch 8, wobei
der Bestrahlungsraum umfasst:
- eine bewegbare Gantry (21), mit der der Partikelstrahl aus verschiedenen einstellbaren Richtungen auf das zu bestrahlende Objekt (47) richtbar ist,
- einen entfernbaren Boden (41) im Bestrahlungsraum (31).

10. Verfahren zum Betreiben einer Positioniervorrichtung (45) zum Positionieren eines Patienten (49) in einer medizinischen Diagnostik- und/oder Therapieanlage (10), wobei die Positioniervorrichtung (45) eine Patientenlagerungsvorrichtung (47), auf der ein Patient (49) gelagert wird, und einen Roboterarm (51) mit mehreren Bewegungsachsen (53, 55), mit dem die Patientenlagerungsvorrichtung (47) positioniert wird, aufweist, das Verfahren umfassend:
- Bereitstellen eines regulären Betriebsmodus (81), in dem die Patientenlagerungsvorrichtung (47) vollautomatisch an einer durch eine Steuerungsvorrichtung vorgegebenen Position positioniert wird,
- Bereitstellen eines manuellen Betriebsmodus (83), in dem die Position der Patientenlagerungsvorrichtung (47) manuell änderbar ist,
- Wechsel von dem regulären Betriebsmodus (81) in den manuellen Betriebsmodus (83) bei Vorliegen einer Wechselbedingung (85).

11. Verfahren nach Anspruch 10, wobei
in den manuellen Betriebsmodus (83) die Position der Patientenlagerungsvorrichtung (47) um eine vertikale Drehachse (54, 56) änderbar ist.

12. Verfahren nach Anspruch 10 oder 11, wobei
die Wechselbedingung (85) automatisch dann vorliegt, wenn eine Fehlerbedingung (87) im regulären Betriebsmodus (81) festgestellt wird.

13. Verfahren nach einem der Ansprüche 10 bis 12, wobei
die Wechselbedingung (85) nach manueller Betätigung (87) einer Umschaltvorrichtung (57, 59) vorliegt.

14. Verfahren nach Anspruch 13, wobei
das Betätigen der Umschaltvorrichtung (59) eine mechanische Entsperrung einer der Bewegungsachsen (55) des Roboterarms (51) bewirkt.
